# EUROPEAN PATENT SPECIFICATION

(11) **EP 1 697 313 B1**
(45) Date of publication and mention of the grant of the patent: **16.09.2015**
(21) Application number: 04806260.8
(22) Date of filing: 17.12.2004
(51) Int. Cl.: C07C 269/02, C07C 269/04, C07C 269/06, C07C 269/08, C07C 271/44, A61K 31/27

(54) **PROCESSES FOR THE PREPARATION OF AMINOALKYL PHENYLCARBAMATES**
VERFAHREN ZUR HERSTELLUNG VON PHENYLCARBAMINSÄUREAMINOALKYLESTERN
PROCÉDÉS POUR PRÉPARER DES PHENYLCARBAMATES D'AMINOALKYLE

(30) Priority: 24.12.2003 GB 0329970
(43) Date of publication of application: 06.09.2006
(73) Proprietor: Generics [UK] Limited, Potters Bar Hertfordshire EN6 1AG (GB)
(72) Inventor: GAITONDE, Abhay, Naupada, Thane, Maharashtra (IN); MANGLE, Mangesh, Kalyan, Thane, Maharashtra (IN); PAWAR, Sanjay, R., Bhandup (West), Mumbai, Maharashtra (IN)
(74) Representative: Elend, Almut Susanne
(86) International application number: PCT/GB2004/050042
(87) International publication number: WO 2005/061446

(56) References cited:
- EP-B1- 0 193 926
- WO-A-00/14057
- WO-A-2004/037771
- WO-A2-03/101917
- US-A- 4 948 807
- CISZEWSKA G ET AL: "SYNTHESIS OF TRITIUM, DEUTERIUM, AND CARBON-14 LABELED (S)-N-ETHYL-N-METHYL-3-[1-(DIMETHYLAMINO)E THYL]CARBAMIC ACID, PHENYL ESTER,(L)-2,3-DIHYDROXYBUTANEDIOIC ACID SALT (SDZ ENA 713 HTA), AN INVESTIGATIONAL DRUG FOR THE TREATMENT OF ALZHEIMER'S DISEASE" JOURNAL OF LABELLED COMPOUNDS AND RADIOPHARMACEUTICALS, SUSSEX, GB, vol. 39, no. 8, 1997, pages 651-668, XP002269029 ISSN: 0362-4803
- DATABASE BEILSTEIN [Online] XP002327188 accession no. RID 8695727
- DATABASE BEILSTEIN [Online] XP002327189 accession no. RID 816415
- DATABASE BEILSTEIN [Online] XP002327190 accession no. RID 945119
- DATABASE BEILSTEIN [Online] XP002327191 accession no. RID 771876
- DATABASE BEILSTEIN [Online] XP002327192 accession no. BRN 3652483
- DATABASE BEILSTEIN [Online] XP002327193 accession no. BRN 3646129
- DATABASE BEILSTEIN [Online] XP002327194 accession no. BRN 3644242
- DATABASE BEILSTEIN [Online] XP002327195 accession no. BRN 3340624
- DATABASE BEILSTEIN [Online] XP002327196 accession no. BRN 3331553
- DATABASE BEILSTEIN [Online] XP002327197 accession no. BRN 3283495
- DATABASE BEILSTEIN [Online] XP002327198 accession no. BRN 3205503
- DATABASE BEILSTEIN [Online] XP002327199 accession no. BRN 3101218
- DATABASE BEILSTEIN [Online] XP002327200 accession no. BRN 9449166
- DATABASE BEILSTEIN [Online] XP002327201 accession no. BRN 9030136

## Description

### Technical field

The present invention relates to a process for preparing an aminoalkyl phenylcarbamate (4), comprising the steps of converting a hydroxy phenyl ketone (2) to a phenylcarbamate ketone (3), and converting the phenylcarbamate ketone (3) to the desired aminoalkyl phenylcarbamate (4). Optionally the aminoalkyl phenylcarbamate (4) may be resolved into its enantiomers and/or converted into a pharmaceutically acceptable acid addition salt.

The invention allows access to aminoalkyl phenylcarbamates (4) and pharmaceutically acceptable salts prepared by the above process, in particular, to rivastigmine (1) and rivastigmine hydrogen tartrate (9). The aminoalkyl phenylcarbamates (4) may be comprised in pharmaceutical compositions.

The aminoalkyl phenylcarbomates (4), prepared by a process of the present invention, may be used in a method of inhibiting acetylcholine esterase or treating dementia, senile dementia, Alzheimer's disease, Huntington's chorea, tardive dyskinesias, hyperkinesia, a confusion disorder, ataxia, Friedrich's ataxia, Down's syndrome, mania or an acute confusion disorder.

### Background art

Certain aminoalkyl phenylcarbamates are selective acetylcholine esterase inhibitors and are therefore potentially useful as pharmaceuticals for the treatment of brain disorders such as dementia, Alzheimer's disease, Huntington's chorea, tardive dyskinesias, confusion disorders and ataxia.

One such compound, the hydrogen tartrate salt of (S)-N-ethyl,methyl-3-[1-(dimethylamino)ethyl]phenylcarbamate (rivastigmine (1)), is marketed as a pharmaceutical for the treatment of dementia of the Alzheimer's type.

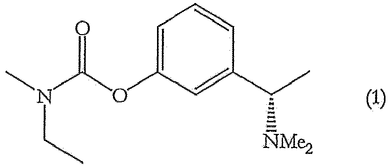

Processes for the preparation of these types of phenylcarbamates are described in US 4 948 807, US 5 602 176, WO 2004/037771, Journal of Labelled Compounds and Radiopharmaceuticals (1997, vol. 39(8), pages 651-668), and Beilstein nos. RID 8695727, RID 816415 and RID 771876. Aminoalkyl phenylcarbamates are also described in Beilstein nos. BRN 3652483, BRN 3646129, BRN 3644242, BRN 3340624, BRN 3331553, BRN 3283495, BRN 3205503, BRN 9449166 and BRN 9030136. Other types of carbamates are described in WO 00/14057, and Beilstein nos. RID 945119 and BRN 3101218.

However, these known processes are not satisfactory, particularly for industrial scale manufacture, as they have been found to involve zwitterionic intermediates, which are very water soluble and need to be isolated by concentration of the aqueous solvent. Other disadvantages of the processes are that they tend to leave large amounts of sulphated ash as residues and that pyrophoric and hazardous reagents such sodium hydride are employed.

### Summary of the invention

According to the present invention, there is provided a process for preparing an aminoalkyl phenylcarbamate of formula (4): 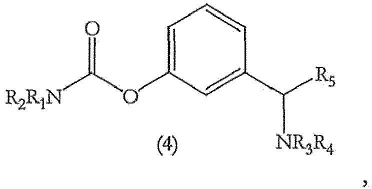 wherein R₁, R₂, R₃, R₄ and R₅ are the same or different, each being hydrogen or an alkyl, alkenyl, alkynyl, aryl, arylalkyl, arylalkenyl, arylalkynyl, alkylaryl, alkenylaryl or alkynylaryl group, each of which may optionally be substituted, and each of which may optionally include one or more heteroatoms N, O or S in its carbon skeleton, or wherein -NR₁R₂ together and/or -NR₃R₄ together form a cycloheteroalkyl, cycloheteroalkenyl, heteroaryl, arylcycloheteroalkyl, arylcycloheteroalkenyl, alkylheteroaryl, alkenylheteroaryl or alkynylheteroaryl group, each of which may optionally be substituted, and each of which may optionally include one or more further heteroatoms N, O or S in its carbon skeleton,
the process comprising the steps of:
(a) converting a hydroxy phenyl ketone of formula (2) to a phenylcarbamate ketone of formula (3): 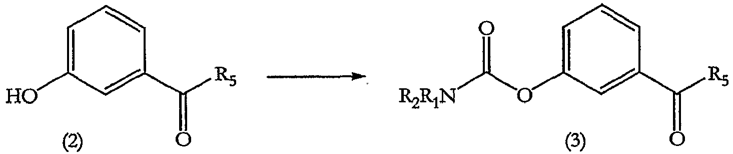 and
(b) converting the phenylcarbamate ketone of formula (3) to the aminoalkyl phenylcarbamate of formula (4):

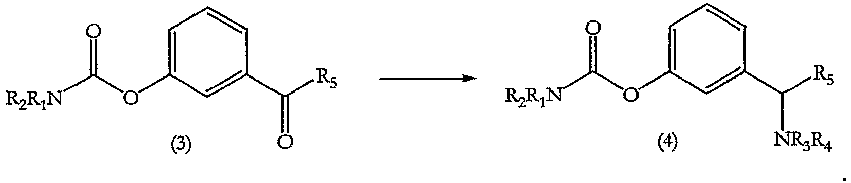

For the purposes of the present invention, where a combination of groups is referred to as one moiety, for example, arylalkyl, arylalkenyl, arylalkynyl, alkylaryl, alkenylaryl or alkynylaryl, the last mentioned group contains the atom by which the moiety is attached to the rest of the molecule. A typical example of an arylalkyl group is benzyl.

For the purposes of this invention, an optionally substituted group may be substituted with one or more of -F, -Cl, -Br, -I, -CF₃, -CCl₃, -CBr₃, -CI₃, -OH, -SH, -NH₂, -CN, -NO₂, -COOH, -R₇-O-R₈, -R₇-S-R₈, -R₇-SO-R₈, -R₇-SO₂-R₈, -R₇-N(R₈)₂, -R₇-N(R₈)₃⁺, -R₇-P(R₈)₂, -R₇-Si(R₈)₃, -R₇-CO-R₈, -R₇-CO-OR₈, -R₇O-CO-R₈, -R₇-CO-N(R₈)₂, -R₇-NR₈-CO-R₈, -R₇O-CO-OR₈, -R₇O-CO-N(R₈)₂, -R₇-NR₈-CO-OR₈, -R₇-NR₈-CO-N(R₈)₂, -R₇-CS-R₈, -R₇-CS-OR₈, -R₇O-CS-R₈, -R₇-CS-N(R₈)₂, -R₇-NR₈-CS-R₈, -R₇O-CS-OR₈, -R₇O-CS-N(R₈)₂, -R₇-NR₈-CS-OR₈, -R₇-NR₈-CS-N(R₈)₂ or -R₈. In this context, -R₇- is independently a chemical bond, a C₁-C₁₀ alkylene, C₁-C₁₀ alkenylene or C₁-C₁₀ alkynylene group. -R₈ is independently hydrogen, an unsubstituted C₁-C₆ alkyl group or an unsubstituted C₆-C₁₀ aryl group. Optional substituent(s) are not taken into account when calculating the total number of carbon atoms in the parent group substituted with the optional substituent(s). Preferably a substituted group comprises 1, 2 or 3 substituents, more preferably 1 or 2 substituents, and even more preferably 1 substituent.

Any optional substituent may be protected. Suitable protecting groups for protecting optional substituents are known in the art, for example from "Protective Groups in Organic Synthesis" by T.W. Greene and P.G.M. Wuts (Wiley-Interscience, 2nd edition, 1991).

In a preferred embodiment, none of R₁, R₂, R₃, R₄ and R₅ is substituted. In another preferred embodiment, none of R₁, R₂, R₃, R₄ and R₅ includes any heteroatoms in its carbon skeleton.

In another preferred embodiment, R₁ and R₅ are an alkyl group.

In another preferred embodiment, R₁, R₂, R₃, R₄ and R₅ are the same or different, each being hydrogen or an alkyl group. Preferably, R₁, R₂, R₃, R₄ and R₅ are the same or different, each being hydrogen or a C₁₋₆ alkyl group. More preferably, R₁, R₂, R₃, R₄ and R₅ are the same or different, each being hydrogen or a C₁₋₃ alkyl group. Even more preferably, R₁, R₂, R₃, R₄ and R₅ are the same or different, each being hydrogen, methyl or ethyl. In one embodiment, at least one of R₁, R₂, R₃, R₄ and R₅ is not hydrogen.

In another preferred embodiment, -NR₁R₂ together and/or -NR₃R₄ together form a ring.

In another preferred embodiment, R₁ and R₂ are the same or different, each being hydrogen or a C₁₋₆ alkyl group. Preferably, R₁ and R₂ are the same or different, each being hydrogen or a C₁₋₃ alkyl group. More preferably, R₁ and R₂ are the same or different, each being hydrogen, methyl or ethyl.

In another preferred embodiment, R₃ and R₄ are the same or different, each being hydrogen or a C₁₋₆ alkyl group, or -NR₃R₄ together form a cycloheteroalkyl group, which may optionally be substituted, and which may optionally include one or more further heteroatoms N, O or S in its carbon skeleton. Preferably, R₃ and R₄ are the same or different, each being hydrogen or a C₁₋₆ alkyl group, or -NR₃R₄ together form a C₁₋₅ cycloheteroalkyl group, which may optionally include one further heteroatom N, O or S in its carbon skeleton. More preferably, R₃ and R₄ are the same or different, each being hydrogen or a C₁₋₃ alkyl group, or -NR₃R₄ together form 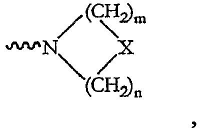 wherein X is CH₂, CH-OH, CO, NH, NOH, O, S, SO or SO₂; m and n are independently 0, 1, 2, 3, 4 or 5, and the sum of m and n is 1, 2, 3, 4 or 5. Even more preferably, R₃ and R₄ are the same or different, each being hydrogen, methyl or ethyl.

In another preferred embodiment, R₅ is hydrogen, a C₁₋₆ alkyl group, or -(CH₂)_{P}-Y-C₆H₄-Z, wherein Y is CH₂, CH-OH, CO, NH, NOH, O, S, SO or SO₂; Z is -R₆, -OR₆, -F, -Cl, -Br, -I, -CF₃, -NO₂, -N(R₆)₂ or -CN with each R₆ independently being hydrogen or a C₁₋₄ alkyl group; and p is 0, 1, 2 or 3. Preferably, R₅ is hydrogen or a C₁₋₃ alkyl group. More preferably, R₅ is hydrogen, methyl or ethyl.

In a preferred embodiment, step (a) is carried out using carbamoyl chloride R₁R₂N-COCI, wherein R₁ and R₂ are as defined above. Preferably, step (a) is carried out in the presence of a base. Preferably, step (a) is carried out in the presence of K₂CO₃ or triethylamine, more preferably step (a) is carried out in the presence of K₂CO₃.

Alternatively, step (a) may be carried out using isocyanate R₁N=C=O, wherein R₁ is as defined above and R₂ is hydrogen. Alternatively still, step (a) may be carried out using anhydride R₁R₂N-CO-O-CO-NR₁R₂, wherein R₁ and R₂ are as defined above.

In a preferred embodiment, step (b) is carried out using amine HNR₃R₄, wherein R₃ and R₄ are as defined above.

Preferably, step (b) is carried out by reductive amination of the carbonyl group of ketone (3) with amine HNR₃R₄. The reductive amination may be carried out in the presence of any suitable reducing agent, such as hydrogen and a hydrogenation catalyst such as nickel; NaBH₄; NaCNBH₃; zinc and HCl; Fe(CO)₅ and alcoholic KOH; PhSeH; or HCO₂H. Preferably, the reductive amination is carried out in the presence of NaCNBH₃.

Alternatively, step (b) may be carried out by reduction of the carbonyl group of ketone (3) to yield a phenylcarbamate alcohol, followed by conversion of the phenylcarbamate alcohol to a phenylcarbamate bromide (or a phenylcarbamate bearing another suitable leaving group, such as a phenylcarbamate iodide, chloride, mesylate or tosylate), followed in turn by the conversion of the phenylcarbamate bromide (or iodide, chloride, mesylate or tosylate) into the desired amine (4).

In a preferred embodiment, the process of the present invention further comprises the step of:
(c) resolving the aminoalkyl phenylcarbamate of formula (4) to obtain an aminoalkyl phenylcarbamate of formula (5) or (10): 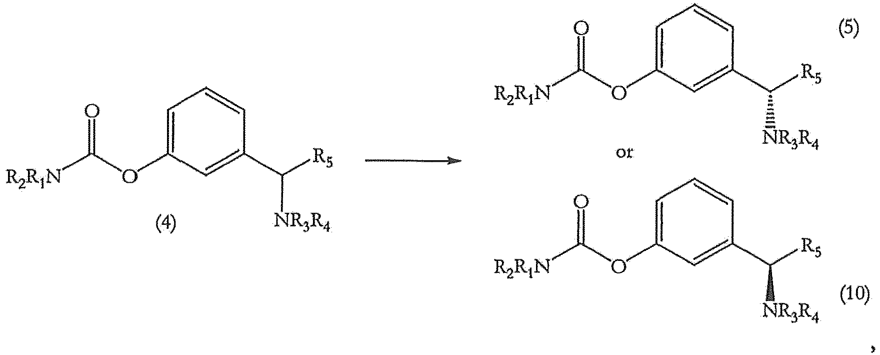 wherein R₁, R₂, R₃, R₄ and R₅ are as defined above, and
   wherein the isomer of formula (5) or (10) obtained comprises less than 5% of other isomers of the same compound.

Preferably, the aminoalkyl phenylcarbamate of formula (4) is resolved to obtain a substantially enantiomerically pure aminoalkyl phenylcarbamate of formula (5). Alternatively, the aminoalkyl phenylcarbamate of formula (4) is resolved to obtain a substantially enantiomerically pure aminoalkyl phenylcarbamate of formula (10).

Preferably, the resolution is carried out using an enantiomerically pure acid to crystallise one isomer of aminoalkyl phenylcarbamate (4) in preference to one or more other isomers. More preferably, the resolution is carried out using (+)-di-Op-toluyl-tartaric acid (DPTTA) to crystallise one isomer of aminoalkyl phenylcarbamate (4) in preference to one or more other isomers.

Alternatively, instead of or in addition to carrying out resolution step (c), step (b) can be carried out stereoselectively to obtain an enantiomerically enriched or substantially enantiomerically pure aminoalkyl phenylcarbamate of formula (5) or (10).

The compounds prepared by the present invention contain at least one chiral centre. The compounds therefore exist in at least two isomeric forms. The present invention encompasses the preparation of racemic mixtures of the compounds as well as enantiomerically enriched and substantially enantiomerically pure isomers of the compounds. For the purposes of this invention, a "substantially enantiomerically pure" isomer of a compound comprises less than 5% of other isomers of the same compound, preferably less than 3%, more preferably less than 2%, more preferably less than 1%, and even more preferably less than 0.5%. Thus in a preferred embodiment, the aminoalkyl phenylcarbamate prepared by a process of the present invention is a substantially enantiomerically pure isomer of formula (5) or (10) comprising less than 5% of other isomers of the same compound, preferably less than 3%, more preferably less than 2%, more preferably less than 1 %, and even more preferably less than 0.5%.

In a preferred embodiment, the process of the present invention further comprises the step of forming a salt of formula (4s), (5s) or (10s) of the aminoalkyl phenylcarbamate of formula (4), (5) or (10): 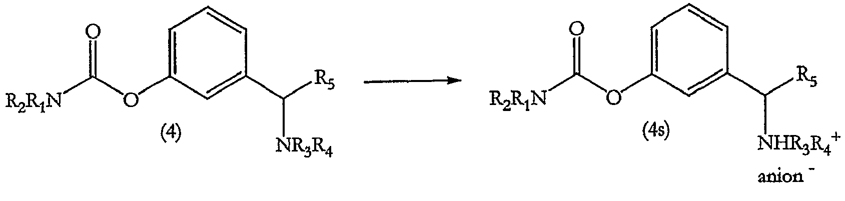 or 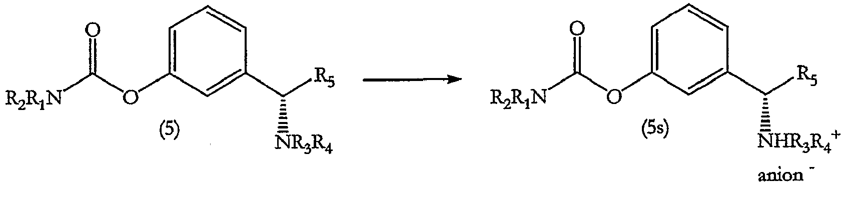 or 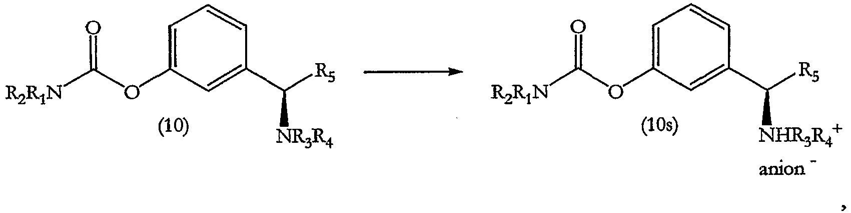 wherein R₁, R₂, R₃, R₄ and R₅ are as defined above, and wherein anion⁻ is the anion of any pharmaceutically acceptable acid.

Preferably, the anion is an anion of hydrofluoric, hydrochloric, hydrobromic, hydroiodic, nitric, perchloric, sulphuric, phosphoric, propionic, butyric, glycolic, lactic, mandelic, citric, acetic, benzoic, salicylic, succinic, malic or hydroxysuccinic, tartaric, fumaric, maleic, hydroxymaleic, mucic or galactaric, gluconic, pantothenic, pamoic, methanesulphonic, trifluoromethanesulphonic, ethanesulphonic, 2-hydroxyethanesulphonic, benzenesulphonic, toluene-p-sulphonic, naphthalene-2-sulphonic, camphorsulphonic, ornithinic, glutamic or aspartic acid. More preferably, the anion is an anion of acetic, salicylic, fumaric, phosphoric, sulphonic, maleic, succinic, citric, tartaric, propionic or butyric acid. Even more preferably, the anion is an anion of tartaric acid.

In a preferred embodiment, the process of the present invention is substantially as hereinafter described with reference to the scheme of Figure One, wherein R₁, R₂, R₃, R₄ and R₅ are the same or different, each being hydrogen or an alkyl, alkenyl, alkynyl, aryl, arylalkyl, arylalkenyl, arylalkynyl, alkylaryl, alkenylaryl or alkynylaryl group, each of which may optionally be substituted, and each of which may optionally include one or more heteroatoms N, O or S in its carbon skeleton, or wherein -NR₂R₂ together and/or -NR₃R₄ together form a cycloheteroalkyl, cycloheteroalkenyl, heteroaryl, arylcycloheteroalkyl, arylcycloheteroalkenyl, alkylheteroaryl, alkenylheteroaryl or alkynylheteroaryl group, each of which may optionally be substituted, and each of which may optionally include one or more further heteroatoms N, O or S in its carbon skeleton. Preferably, the process of the present invention is as hereinafter described with reference to the scheme of Figure Two.

In a preferred embodiment, less than 10% sulphated ash is produced as by-product when carrying out the process of the present invention, more preferably less than 5%, more preferably less than 1%, and even more preferably less than 0.1%.

In a preferred embodiment, aminoalkyl phenylcarbamate (4) is prepared in substantially pure form when carrying out the process of the present invention. For the purposes of the present invention, the aminoalkyl phenylcarbamate (4) is "substantially pure", if it comprises less than 10% impurities, preferably less than 5%, more preferably less than 1%, and even more preferably less than 0.1%.

In a preferred embodiment, step (a) is carried out in the substantial absence of NaH. Preferably, step (a) is carried out in the substantial absence of any pyrophoric reagents.

For the purposes of the present invention, step (a) is carried out in the "substantial absence of NaH", if the reaction mixture comprises less than 0.5 molar equivalents of NaH, more preferably less than 0.2, more preferably less than 0.1, and even more preferably less than 0.01, wherein molar equivalents of NaH are in respect of the hydroxy phenyl ketone of formula (2). The term "substantial absence of any pyrophoric reagents" shall be construed accordingly.

In a preferred embodiment, the process of the present invention is carried out on an industrial scale. This means that the aminoalkyl phenylcarbamate (4), (5) or (10), or a pharmaceutically acceptable salt thereof, may be manufactured in batches of 5kg or more, 10kg or more, or even 30kg or more.

In a preferred embodiment, substantially no zwitterionic intermediates are formed and/or isolated when carrying out the process of the present invention.

For the purposes of the present invention, "no zwitterionic intermediates are formed", if the reaction mixture comprises less than 5% by weight zwitterionic intermediates, preferably less than 2% by weight, more preferably less than 1% by weight, and even more preferably less than 0.5% by weight. The term "no zwitterionic intermediates are isolated" shall be construed accordingly.

An aminoalkyl phenylcarbamate of formula (4), (5) or (10), or a pharmaceutically acceptable salt thereof, may be prepared by a process of the present invention.

In a preferred embodiment, the aminoalkyl phenylcarbamate is rivastigmine (1) or a pharmaceutically acceptable salt thereof. Hence, rivastigmine (1) or a pharmaceutically acceptable salt thereof, may be prepared by a process of the present invention.

In a preferred embodiment, the aminoalkyl phenylcarbamate is rivastigmine hydrogen tartrate (9). Hence rivastigmine hydrogen tartrate (9) may be prepared by a process of the present invention.

The aminoalkyl phenylcarbamate may be used for inhibiting acetylcholine esterase or for use in the treatment of dementia, senile dementia, Alzheimer's disease, Huntington's chorea, tardive dyskinesias, hyperkinesia, a confusion disorder, ataxia, Friedrich's ataxia, Down's syndrome, mania or an acute confusion disorder. Preferably, the aminoalkyl phenylcarbamate may be used for use in the treatment of dementia, Alzheimer's disease, Huntington's chorea, tardive dyskinesias, a confusion disorder or ataxia.

An aminoalkyl phenylcarbamate prepared by a process of the present invention may be used in a pharmaceutical composition, comprising the aminoalkyl phenylcarbamate and a pharmaceutically acceptable excipient or carrier.

The pharmaceutical composition may be used for inhibiting acetylcholine esterase or for use in the treatment of dementia, senile dementia, Alzheimer's disease, Huntington's chorea, tardive dyskinesias, hyperkinesia, a confusion disorder, ataxia, Friedrich's ataxia, Down's syndrome, mania or an acute confusion disorder. Preferably, the pharmaceutical composition may be used for use in the treatment of dementia, Alzheimer's disease, Huntington's chorea, tardive dyskinesias, a confusion disorder or ataxia.

An aminoalkyl phenylcarbamate, prepared by a process of the present invention, may be used in a method of inhibiting acetylcholine esterase, comprising administering a therapeutically effective amount of the aminoalkyl phenylcarbamate to a patient in need thereof.

An aminoalkyl phenylcarbamate, prepared by a process of the present invention, may also be used in a method of treating dementia, senile dementia, Alzheimer's disease, Huntington's chorea, tardive dyskinesias, hyperkinesia, a confusion disorder, ataxia, Friedrich's ataxia, Down's syndrome, mania or an acute confusion disorder, comprising administering a therapeutically effective amount of the aminoalkyl phenylcarbamate to a patient in need thereof. Preferably the method is for treating dementia, Alzheimer's disease, Huntington's chorea, tardive dyskinesias, a confusion disorder or ataxia.

A use of an aminoalkyl phenylcarbamate prepared by a process of the present invention is in the manufacture of a medicament for inhibiting acetylcholine esterase.

A further use of an aminoalkyl phenylcarbamate prepared by a process of the present invention is in the manufacture of a medicament for the treatment of dementia, senile dementia, Alzheimer's disease, Huntington's chorea, tardive dyskinesias, hyperkinesia, a confusion disorder, ataxia, Friedrich's ataxia, Down's syndrome, mania or an acute confusion disorder. Preferably the medicament is for the treatment of dementia, Alzheimer's disease, Huntington's chorea, tardive dyskinesias, a confusion disorder or ataxia.

### Brief description of the drawings

The present invention will now be described by way of example with reference to the accompanying drawings in which:
Figure One shows a general reaction scheme illustrating the process of the present invention.
Figure Two shows a reaction scheme illustrating a preferred embodiment of the process of the present invention.
Figure Three shows a reaction scheme illustrating an alternative embodiment of the process of the present invention.

### Detailed description of the invention

The inventors have found that the preparation of aminoalkyl phenylcarbamates is greatly improved by the process outlined in the scheme of Figure One, wherein R₁, R₂, R₃, R₄ and R₅ are the same or different and can be as previously defined, preferably an alkyl group. Alternatively, R₁-R₂ and/or R₃-R₄ together form a ring.

The process outlined in the scheme of Figure One is short, utilises readily available hydroxy phenyl ketones (2) as starting materials and does not require any hazardous chemical reagents. Each step of the process is high yielding and affords products of very high purity.

Therefore the current invention provides a process for the preparation of aminoalkyl phenylcarbamates by a process comprising the steps specified in the scheme of Figure One.

A preferred embodiment of the invention is a process for the preparation of rivastigmine hydrogen tartrate (9), as outlined in the scheme of Figure Two.

The process of the invention, embodied by the process depicted in the scheme of Figure Two, avoids the problems of prior art processes as no zwitterionic intermediates are involved and consequently the intermediates can be efficiently isolated by extraction. The sulphated ash residues in the process of the invention are less than 0.1%, whereas in the prior art processes the sulphated ash residues can be greater than 20%. In addition, the process according to the invention avoids the use of pyrophoric and hazardous reagents such as sodium hydride and preferably replaces this reagent with less expensive and safer reagents such as potassium carbonate (see, for example, step (a) in the scheme of Figure Two).

Figure Three shows a reaction scheme illustrating an alternative embodiment of the process of the present invention.

The invention allows access to rivastigmine (1) and/or its pharmaceutically acceptable salts when prepared by a process according to the current invention. In a preferred embodiment rivastigmine hydrogen tartrate (9) is prepared by a process according to the invention.

The processes outlined in the schemes of Figures Two and Three are examples of procedures comprising the process of the current invention and detailed procedures for these processes are found in the experimental procedures outlined below.

### Experimental details - Figure Two

### 3-O-((N-ethyl,methyl)carbamoyl)acetophenone (7)

Acetone (1000ml) was charged in a flask equipped with a reflux condenser and mechanical stirrer. 3'-Hydroxyacetophenone (6) (125g, 0.919mol) was added with stirring at ambient temperature. This gave a yellow clear coloured solution. Potassium carbonate (381g, 2.757mol, 3eq) was added in one lot followed by addition of ethyl methyl carbamoyl chloride (EMCC, 167.5g, 1.379mol, 1.5eq) also in one lot. Acetone (250ml) used to rinse the addition funnel was added to the reaction. The reaction mixture was then heated to reflux (55°C). After 4 hours, potassium carbonate (25.5g, 0.18mol, 0.2eq) was added in one lot followed by acetone (92ml, 0.74 volumes). One hour after this addition, potassium carbonate (12.75g, 0.09mol, 0.1eq) was added in one lot. The reaction mass was allowed to cool to about 35°C and filtered though a sintered funnel under vacuum. The solid was discarded and the mother liquor was concentrated to obtain crude ketone (7) as a red coloured liquid.
Yield: 229g, 112.74%. HPLC purity: 96.71%. Sulphated ash: 0.4%. ¹H NMR: δ 1.41-1.56 (2 x t, 3H, N-CH₂CH₃), 8 2.6 (s, 3H, COCH₃), δ 3.00 and 3.09 (2 x s, 3H, N-CH₃), δ 3.38-3.53 (2 x q, 2H, N-CH₂CH₃), δ 7.32 (d, 1H, Ar-H), δ 7.46 (t, 1H, Ar-H), δ 7.69 (s, 1H, Ar-H), 8 7.78 (d, 1H, Ar-H).

### Ethylmethylcarbamic acid 3-[1-(dimethylamino)ethylphenyl esther (8)

A 2M solution of dimethylamine in methanol (methanolic DMA, 3.061, 6.12mol, 6eq) was cooled to about 5°C in a 51 flask equipped with a thermometer pocket and mechanical stirrer. Concentrated hydrochloric acid (conc. HCl, 213ml, 2.04mol, 2eq) was added over 30 minutes. The temperature rose to about 8°C. Ketone (7) (225g, 1.02mol, 1eq) was added as one lot to the reaction mixture at 8-10°C. NaCNBH₃ (44.867g, 0.714mol, 0.7eq) was added in one lot and the reaction mixture was brought up from 8-10°C to 24-26°C. The reaction mass was stirred for 48 hours after and filtered though a sintered funnel under vacuum. The solid was discarded and the mother liquor was concentrated to obtain 561g. Water (2.251) was added. The pH was about 8 (measured using pH paper). The aqueous solution was acidified with 1:9 dilute HCl (650ml) to pH 2. The acidified aqueous phase was extracted with ethyl acetate (3 x 1.1251, 3 x 5 volumes) to remove the unreacted starting material. The aqueous phase was then basified with saturated sodium carbonate solution (220ml) to pH 9. This basified aqueous layer was extracted with ethyl acetate (3 x 1.1251, 3 x 5 volumes). These three ethyl acetate layers were mixed and concentrated to obtain crude amine (8) as red coloured liquid.
Yield: 141g, 55.4%. HPLC purity: 99.09%. Sulphated ash: 0.08%. ¹H NMR: δ 1.16-1.25 (2 x t, 3H, N-CH₂CH₃), δ 1.35 (d, 3H, PhCCH₃), δ 2.2 (s, 6H, N-(CH₃)₂), δ 2.90 and 3.00 (2 x s, 3H, N-CH₃), δ 3.21-3.27 (q, 2H, CHCH₃), δ 3.39-3.48 (2 x q, 2H, N-CH₂CH₃), δ 7.0 (d, 1H, Ar-H), δ 7.06 (s, 1H, Ar-H), δ 7.11 (d, 1H, Ar-H), δ 7.28 (t, 1H, Ar-H).

### (S)-N-ethyl,methyl-3-[1-(dimethylamino)ethyl]phenylcarbamate (rivastigmine) (1)

200ml (10 volumes) of a 2:1 v/v mixture of methanol:water was prepared. Of this mixture 160ml was charged in a 500ml flask equipped with a thermometer pocket, reflux condenser, oil bath and magnetic stirrer. Racemic rivastigmine (8) (20g, 0.08mol, 1eq) was charged to the flask in one shot. The remaining 40ml of the methanol:water mixture was used to rinse the funnel used for transfer of (8). (+)-Di-O-p-toluyl-tartaric acid (DPTTA, 32.4g, 0.08mol, 1eq) was charged to the reaction vessel and heating was started. The clear solution was refluxed for 30 minutes at 70°C. The oil bath was then removed and the reaction mixture was allowed to come to 30°C in 20 minutes. The solid that had crystallised out was filtered off under vacuum using a sintered funnel. It was dried on the rotavapor at 50°C and 15mbar pressure for 3 hours to obtain 22.5g of (+)-di-O-p-toluyl tartrate salt of (8). Yield: 22.5g, 42.98%.

### 1^{st} crystallisation

150ml (6.66 volumes) of a 2:1 v/v mixture of methanol:water was charged to a 250ml flask equipped with a thermometer pocket, reflux condenser, oil bath and magnetic stirrer. 22.5g (0.034mol) of the DPTTA salt obtained above was charged to it and heating was started. The clear solution was heated for 20 minutes at 50°C. The oil bath was then removed and the reaction mixture was allowed to come to 30°C in 15 minutes. The solid that had crystallised out was filtered off under vacuum using a sintered funnel. It was dried on the rotavapor at 50°C and 15mbar pressure for 3 hours to obtain 10.274g of once crystallised (+)-di-O-p-toluyl tartrate salt. Yield: 10.274g, 45.66%.

### 2^{nd} crystallisation

122ml (11.87 volumes) of a 2:1 v/v mixture of methanol:water was charged to a 250ml flask equipped with a thermometer pocket, reflux condenser, oil bath and magnetic stirrer. 10.274g (0.016mol) of the DPTTA salt obtained after one crystallisation above was charged to it and heating was started. The clear solution was heated for 30 minutes at 50°C. The oil bath was then removed and the reaction mixture was allowed to come to 30°C in 15 minutes. The solid that had crystallised out was filtered off under vacuum using a sintered funnel. It was dried on the rotavapor at 50°C and 25mbar pressure for 3 hours 45 minutes to obtain 4.174g of twice crystallised (+)-di-O-p-toluyl tartrate salt. Yield: 4.174g, 40.63%.

### 3^{rd} crystallisation

50ml (12 volumes) of a 2:1 v/v mixture of methanol:water was charged to a 100ml flask equipped with a thermometer pocket, reflux condenser, oil bath and magnetic stirrer. 4.174g (0.0064mol) of the DPTTA salt obtained after two crystallisations above was charged to it and heating was started. The clear solution was heated for 30 minutes at 50°C. The oil bath was then removed and the reaction mixture was allowed to come to 30°C in 30 minutes. The solid that had crystallised out was filtered off under vacuum using a sintered funnel. It was dried on the rotavapor at 50°C and 15mbar pressure for 2.5 hours to obtain 2.31g of thrice crystallised (+)-di-O-p-toluyl tartrate salt. Yield: 2.31g, 55.34%.

### Liberation of Rivastigmine (1)

12ml of aqueous 1N NaOH solution (0.12mol) was charged to a flask equipped with a thermometer pocket and ice-bath. The solution was cooled to 5°C and 2g (0.0031mol) of the DPTTA salt obtained after three crystallisations above was charged to it. The ice-bath was removed and after 10 minutes of stirring the solution became turbid (at about 12°C) and was allowed to come to 30°C in about 15 minutes. It was extracted with diethyl ether (3 x 15ml) and the combined organic extracts were dried over sodium sulphate and concentrated to dryness on the rotavapor to obtain 750mg of rivastigmine (1).
Yield: 750mg, 98.2%. [α]_{D} (c=5 methanol) -30.29°. ¹H NMR: δ 1.16-1.25 (2 x t, 3H, N-CH₂CH₃), δ 1.35 (d, 3H, PhCCH₃), δ 2.2 (s, 6H, N-(CH₃)₂), δ 2.90 and 3.00 (2 x s, 3H, N-CH₃), δ 3.21-3.27 (q, 2H, CHCH₃), δ 3.39-3.48 (2 x q, 2H, N-CH₂CH₃), δ 7.0 (d, 1H, Ar-H), δ 7.06 (s, 1H, Ar-H), δ 7.11 (d, 1H, Ar-H), δ 7.28 (t, 1H, Ar-H).

### Rivastigmim hydrogen tartrate (9)

Methanol (0.4ml) was taken in a glass vial and L-(+)-tartaric acid (0.204g, 1.36mmol, 1eq) was added to it. It was warmed to 40-45°C to get a clear solution. This was added to a flask containing 0.34g rivastigmine (1) (1.36mmol, 1eq). The clear solution was heated to 55°C for about 5 minutes and kept at 30°C for 2 hours and at 8-10°C overnight. The solvent was evaporated to dryness and the oil so obtained was triturated with hexanes (1 x 5ml, 2 x 10ml) to obtain a solid that was filtered off under vacuum on a sintered funnel under an atmosphere of nitrogen.
Yield: 419mg, 77%. [α]_{D} (c=5 methanol) +5.02°. ¹H NMR: δ 1.05-1.18 (2 x t, 3H, N-CH₂CH₃), δ 1.53 (d, 3H, PhCCH₃), δ 2.54 (s, 6H, N-(CH₃)₂), δ 2.87 and 2.99 (2 x s, 3H, N-CH₃), δ 3.24-3.43 (2 x q, 2H, N-CH₂CH₃), δ 4.22-4.29 (q, 2H, CHCH₃), δ 7.15 (d, 1H, Ar-H), δ 7.22 (s, 1H, Ar-H), δ 7.31 (d, 1H, Ar-H), δ 7.46 (t, 1H, Ar-H).

### Experimental details - Figure Three

### 3-O-((1V-ethyl,methyl)carbamoyl)acetophenone (7)

In a clean and dry 21 4-neck flask was charged triethylamine (593ml) and 3'-hydroxyacetophenone (6) (130g). This reaction mixture was stirred for 5-10 minutes to obtain a clear solution. Then ethyl methyl carbamoyl chloride (EMCC, 34.8g) was added. The reaction mixture was heated to reflux (95°C) and maintained at 95°C for 5 hours. After every 1 hour, the progress of the reaction was monitored by TLC and further EMCC (34.8g) was added. After 5 hours, TLC showed the absence of starting material (6) and the reaction mixture was cooled to 25-30°C. The reaction mixture was filtered through a sintered funnel and the retained solid was washed with triethylamine (25ml). The filtrate was taken up for triethylamine distillation under vacuum at 50°C, remaining traces of triethylamine were removed at high vacuum (10mbar). DCM (650ml) was added to the oil so obtained. The DCM layer was washed with 10% NaOH solution (w/v) (2 x 650ml), washed with demineralized water until the pH of the aqueous layer was between 7-8, dried over Na₂SO₄, and concentrated to dryness to obtain the product (7). Yield: 175g, 82.8%.

### 3-O-((N-ethyl,metbyl)carbamoyl)acetophenone (11)

To a clean and dry 21 4-neck flask was charged THF (850ml) and methanol (85ml). Then ketone (7) (170g) was charged at 26-28°C and the mixture was stirred to obtain a clear solution. This solution was cooled to 20°C. Then NaBH₄ (8.74g) was added over 25 minutes, while the temperature was maintained at 20°C. The reaction mixture was maintained at 24-28°C for a further 2 hours. The progress of the reaction was monitored by TLC every 30 minutes. After 2 hours, TLC showed the absence of starting material (7). The solvent was concentrated under vacuum at 50°C, high vacuum was applied to remove traces of solvent. Demineralized water (1700ml) was added to the crude product at 26-28°C. The aqueous layer was stirred for 5-10 minutes and then extracted with DCM (3 x 510ml). The combined DCM layers were washed with demineralized water (850ml) until the pH of the aqueous layer was between 7-8, dried over Na₂SO₄ (41g), filtered through cotton to remove Na₂SO₄, and concentrated under vacuum at 40°C. High vacuum was applied to remove solvent traces and to yield the product alcohol (11). Yield: 161g, 93.9%.

### 3-O-((N-ethyl,methyl)carbamoyl)acetopbenone (12)

To a clean and dry 21 4-neck flask was charged DCM (465ml) and alcohol (11) (153g) at 26-28°C. PBr₃ (32.7ml) was dissolved in DCM (310ml) and charged to an addition funnel. The reaction mixture was cooled to 0-5°C and PBr₃ solution from the addition funnel was added at 0-5°C to the reaction mixture over 25-30 minutes. The reaction mixture was maintained at 0-5°C, while the progress of the reaction was monitored by TLC after every 30 minutes. After 2 hours, TLC showed the absence of starting material (11). The reaction was quenched by the addition of water (775ml) and the mixture was stirred for 30 minutes at 18°C, pH ∼2-3. Then the DCM layer was separated, washed with 5% Na₂CO₃ solution (w/v) (465ml), washed with demineralized water (3 x 465ml) (pH should be 7-7.5), dried over anhydrous Na₂SO₄ (20g), filtered through cotton to remove Na₂SO₄, and concentrated under vacuum at 40°C. High vacuum was applied to remove solvent traces and to yield the product bromide (12). Yield: 180g, 91.5%.

### Ethylmethylcarbamic acid 3-[1-(dimethylamino)ethyl]phenyl ester (8)

In a clean and dry 51 flask was charged dimethylamine in methanol (methanolic DMA, 3.51) and bromide (12) (175g) at 26-28°C. This reaction mixture was stirred at 26-28°C for 16-20 hours. The progress of the reaction was monitored by TLC. When TLC showed the absence of starting material (12), the reaction mixture was concentrated under vacuum at 40°C. The residue was cool to 26-28°C. Demineralized water (1750ml) was added to the residue and the aqueous mixture stirred for 10-15 minutes. Then the aqueous mixture was cooled to 12-15°C and 10% HCl solution (1400ml) was added slowly, maintaining the temperature below 15°C. Then the aqueous layer was extracted with DCM (3 x 525ml). These first DCM extractions were separated and kept.

The aqueous layer (pH ∼2-3) was cooled to 15-205°C, Na₂CO₃ (219g) was added whilst maintaining the temperature at 15-20°C, and the aqueous layer was extracted with further DCM (3 x 525ml) (pH of aqueous layer ∼10-11).

The first and the further DCM extractions were combined, washed with demineralized water (875ml), dried over Na₂SO₄, filtered to remove Na₂SO₄, and concentrated under vacuum at 40°C. High vacuum was applied to remove solvent traces and to yield the product amine (8). Yield: 109g, 71.2%.

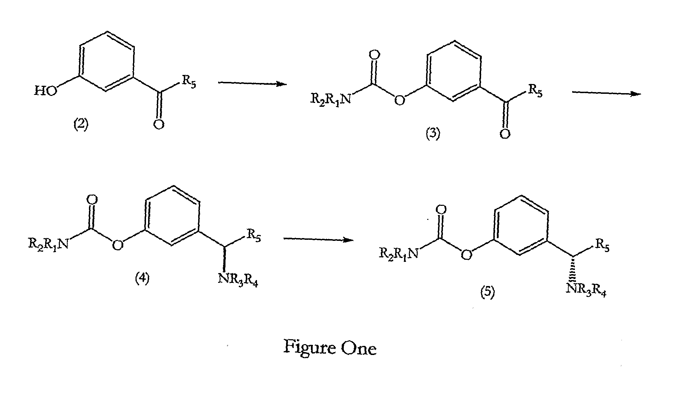

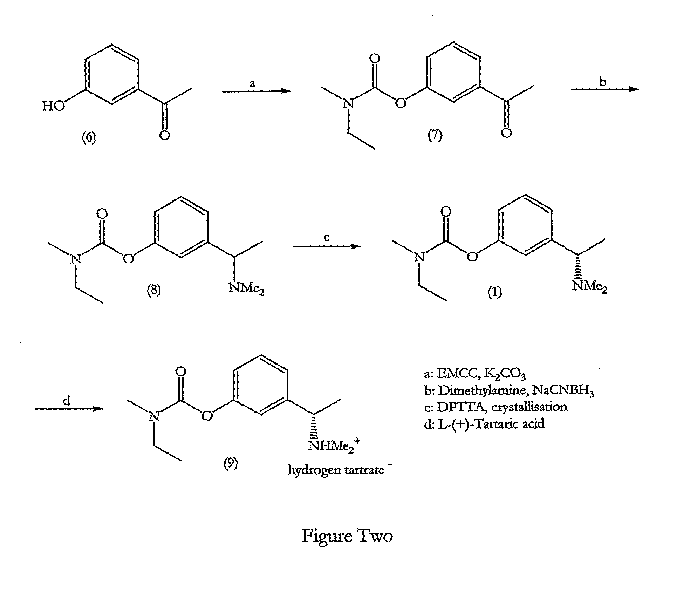

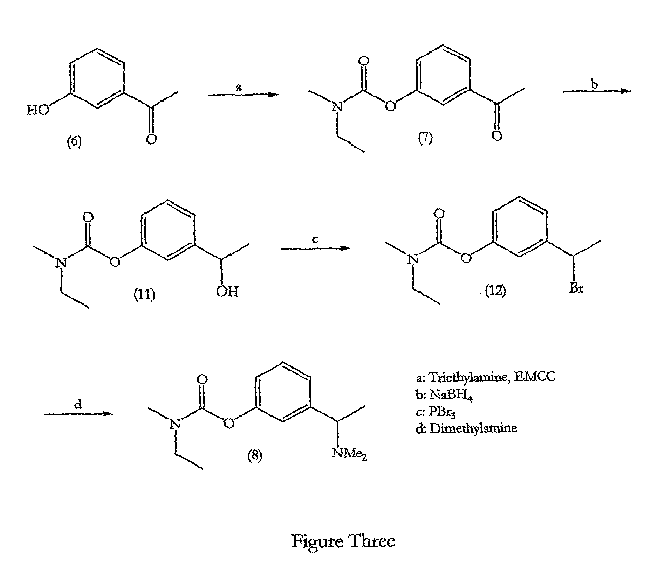

## Claims

1. A process for preparing an aminoalkyl phenylcarbamate of formula (4): 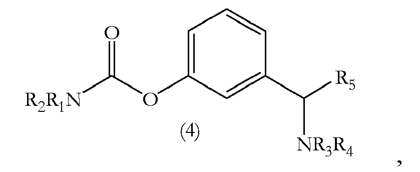 wherein R₁, R₂, R₃, R₄ and R₅ are the same or different, each being hydrogen or an alkyl, alkenyl, alkynyl, aryl, arylalkyl, arylalkenyl, arylalkynyl, alkylaryl, alkenylaryl or alkynylaryl group, each of which may optionally be substituted, and each of which may optionally include one or more heteroatoms N, O or S in its carbon skeleton, or wherein -NR₁R₂ together and/or -NR₃R₄ together form a cycloheteroalkyl, cycloheteroalkenyl, heteroaryl, arylcycloheteroalkyl, arylcycloheteroalkenyl, alkylheteroaryl, alkenylheteroaryl or alkynylheteroaryl group, each of which may optionally be substituted, and each of which may optionally include one or more further heteroatoms N, O or S in its carbon skeleton,
the process comprising the steps of:
(a) converting a hydroxy phenyl ketone of formula (2) to a phenylcarbamate ketone of formula (3): 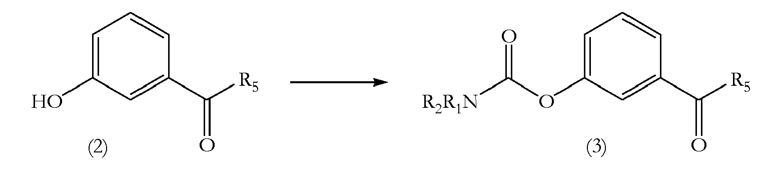 and
(b) converting the phenylcarbamate ketone of formula (3) to the aminoalkyl phenylcarbamate of formula (4):

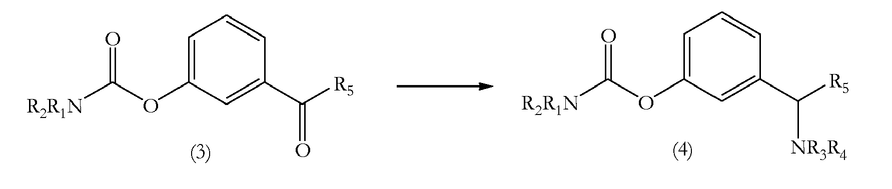

2. A process as claimed in claim 1, wherein:
(a) none of R₁, R₂, R₃, R₄ and R₅ is substituted; and/or
(b) none of R₁, R₂, R₃, R₄ and R₅ includes any heteroatoms in its carbon skeleton.

3. A process as claimed in claim 1 or 2, wherein:
(a) R₁, R₂, R₃, R₄ and R₅ are the same or different, each being hydrogen or an alkyl group; or
(b) R₁, R₂, R₃, R₄ and R₅ are the same or different, each being hydrogen or a C₁₋₆ alkyl group; or
(c) R₁, R₂, R₃, R₄ and R₅ are the same or different, each being hydrogen or a C₁₋₃ alkyl group; or
(d) R₁, R₂, R₃, R₄ and R₅ are the same or different, each being hydrogen, methyl or ethyl.

4. A process as claimed in any one of the preceding claims, wherein R₁, R₂, R₃, R₄ and R₅ are the same or different, each being an alkyl group.

5. A process as claimed in claim 4, wherein R₁ is ethyl and R₂, R₃, R₄ and R₅ are each methyl.

6. A process as claimed in any one of the preceding claims, wherein step (a) is carried out using carbamoyl chloride R₁R₂N-COCl, wherein R₁ and R₂ are defined as in any one of the preceding claims.

7. A process as claimed in claim 6, wherein step (a) is carried out in the presence of a base.

8. A process as claimed in claim 6 or 7, wherein step (a) is carried out in the presence of K₂CO₃ or triethylamine.

9. A process as claimed in any one of the preceding claims, wherein step (b) is carried out using amine HNR₃R₄, wherein R₃ and R₄ are defined as in any one of the preceding claims.

10. A process as claimed in claim 9, wherein step (b) is carried out by reductive amination of the carbonyl group of ketone (3) with amine HNR₃R₄.

11. A process as claimed in claim 9 or 10, wherein step (b) is carried out in the presence of NaCNBH₃.

12. A process as claimed in any one of claims 1 to 9, wherein step (b) is carried out by reduction of the carbonyl group of ketone (3) to yield a phenylcarbamate alcohol, followed by conversion of the phenylcarbamate alcohol to a phenylcarbamate bearing a leaving group, followed in turn by the conversion of the phenylcarbamate bearing a leaving group into the aminoalkyl phenylcarbamate of formula (4).

13. A process as claimed in any one of the preceding claims, further comprising the step of:
(c) resolving the aminoalkyl phenylcarbamate of formula (4) to obtain an aminoalkyl phenylcarbamate of formula (5) or (10): 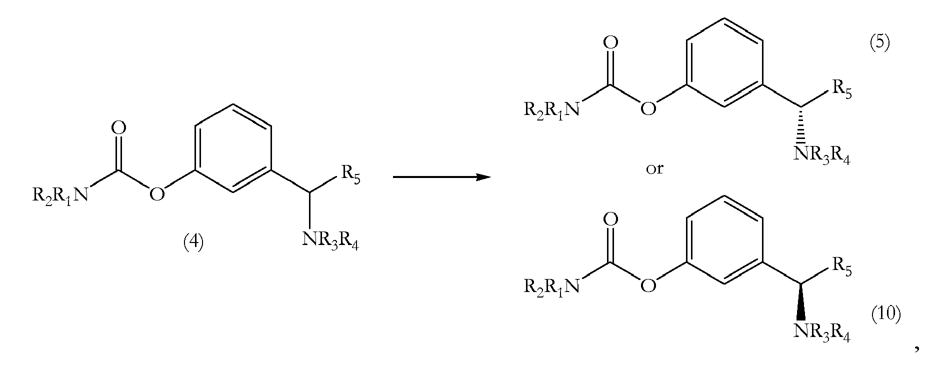 wherein R₁, R₂, R₃, R₄ and R₅ are defined as in any one of the preceding claims, and wherein the isomer of formula (5) or (10) obtained comprises less than 5% of other isomers of the same compound.

14. A process as claimed in claim 13, wherein the resolution is carried out using an enantiomerically pure acid to crystallise one isomer of aminoalkyl phenylcarbamate (4) in preference to one or more other isomers.

15. A process as claimed in claim 13 or 14, wherein the resolution is carried out using (+)-di-O-p-toluyl-tartaric acid (DPTTA) to crystallise one isomer of aminoalkyl phenylcarbamate (4) in preference to one or more other isomers.

16. A process as claimed in any one of the preceding claims, further comprising the step of forming a salt of formula (4s), (5s) or (10s) of the aminoalkyl phenylcarbamate of formula (4), (5) or (10): 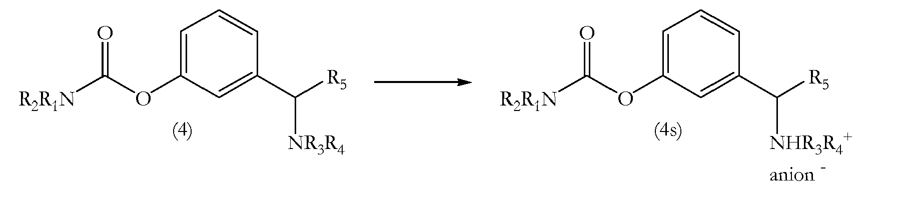 or 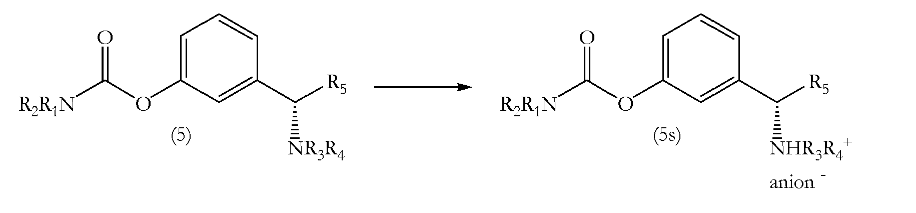 or 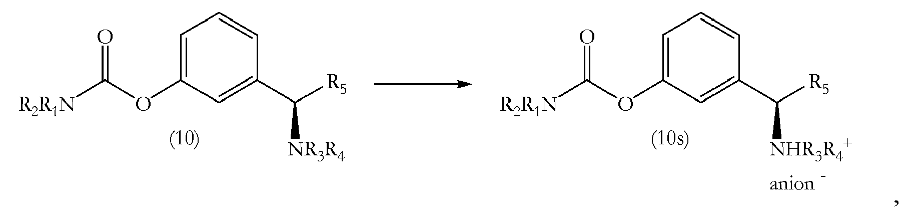 wherein R₁, R₂, R₃, R₄ and R₅ are defined as in any one of the preceding claims, and wherein anion⁻ is the anion of any pharmaceutically acceptable acid.

17. A process as claimed in claim 16, wherein the anion is an anion of:
(a) hydrofluoric, hydrochloric, hydrobromic, hydroiodic, nitric, perchloric, sulphuric, phosphoric, propionic, butyric, glycolic, lactic, mandelic, citric, acetic, benzoic, salicylic, succinic, malic or hydroxysuccinic, tartaric, fumaric, maleic, hydroxymaleic, mucic or galactaric, gluconic, pantothenic, pamoic, methanesulphonic, trifluoromethanesulphonic, ethanesulphonic, 2-hydroxyethanesulphonic, benzenesulphonic, toluene-p-sulphonic, naphthalene-2-sulphonic, camphorsulphonic, ornithinic, glutamic or aspartic acid; or
(b) acetic, salicylic, fumaric, phosphoric, sulphonic, maleic, succinic, citric, tartaric, propionic or butyric acid; or
(c) tartaric acid.

18. A process as claimed in any one of the preceding claims, wherein the reaction mixture in step (a) comprises less than 0.5 molar equivalents of NaH.

19. A process as claimed in any one of the preceding claims, wherein the aminoalkyl phenylcarbamate (4), (5) or (10), or a pharmaceutically acceptable salt thereof, is manufactured in batches of 5kg or more.

20. A process as claimed in any one of the preceding claims, for the preparation of rivastigmine or rivastigmine hydrogen tartrate.

## Patentansprüche

1. Verfahren zum Herstellen eines Aminoalkylphenylcarbamats mit der Formel (4): 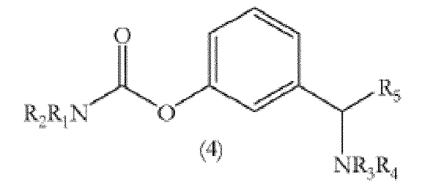 wobei R₁ R₂, R₃, R₄ und R₅ identisch oder verschieden sind, jeweils Wasserstoff oder eine Alkyl-, Alkenyl-, Alkinyl-, Aryl-, Arylalkyl-, Arylalkenyl-, Arylalkinyl-, Alkylaryl-, Alkenylaryl- oder Alkinylarylgruppe sind, die jeweils optional substituiert sein können, und die jeweils optional ein oder mehrere Heteroatome N, O oder S in ihrem Kohlenstoffgerüst aufweisen können, oder wobei -NR₁R₂ gemeinsam und/oder -NR₃R₄ gemeinsam eine Cycloheteroalkyl- , Cycloheteroalkenyl-, Heteroaryl-, Arylcycloheteroalkyl-, Arylcycloheteroalkenyl-, Alkylheteroaryl-, Alkenylheteroaryl- oder Alkynylheteroarylgruppe ausbilden, die jeweils optional substituiert sein können und die jeweils optional ein oder mehrere weitere Heteroatome N, O oder S in ihrem Kohlenstoffgerüst aufweisen können,
wobei das Verfahren ferner die folgenden Schritte umfasst:
(a) Umwandeln eines Hydroxyphenylketons mit der Formel (2) in ein Phenylcarbamatketon mit der Formel (3): 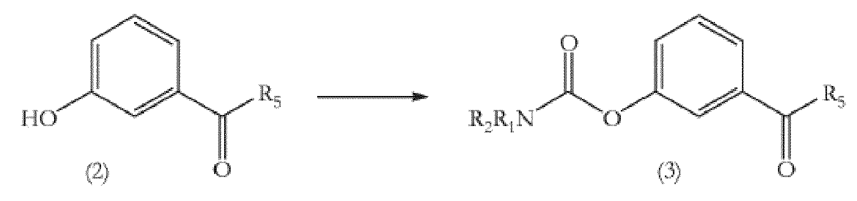 und
(b) Umwandeln des Phenylcarbamatketons mit der Formel (3) in das Aminoalkylphenylcarbamat mit der Formel (4):

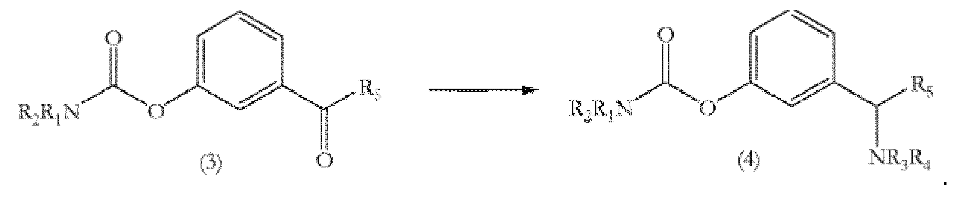

2. Verfahren nach Anspruch 1, wobei:
(a) keiner von R₁, R₂, R₃, R₄ und R₅ substituiert ist; und/oder
(b) keiner von R₁, R₂, R₃, R₄ und R₅ Heteroatome in seinem Kohlenstoffgerüst aufweist.

3. Verfahren nach Anspruch 1 oder 2, wobei:
(a) R₁, R₂, R₃, R₄ und R₅ identisch oder verschieden sind und jeweils Wasserstoff oder eine Alkylgruppe sind; oder
(b) R₁, R₂, R₃, R₄ und R₅ identisch oder verschieden sind und jeweils Wasserstoff oder eine C₁₋₆-Alkylgruppe sind; oder
(c) R₁, R₂, R₃, R₄ und R₅ identisch oder verschieden sind und jeweils Wasserstoff oder eine C₁-₃-Alkylgruppe sind; oder
(d) R₁, R₂, R₃, R₄ und R₅ identisch oder verschieden sind und jeweils Wasserstoff, Methyl oder Ethyl sind.

4. Verfahren nach einem der vorhergehenden Ansprüche, wobei R₁, R₂, R₃, R₄ und R₅ identisch oder verschieden sind und jeweils eine Alkylgruppe sind.

5. Verfahren nach Anspruch 4, wobei R₁ Ethyl ist und R₂, R₃, R₄ und R₅ jeweils Methyl sind.

6. Verfahren nach einem der vorhergehenden Ansprüche, wobei Schritt (a) unter Einsatz von Carbamoylchlorid R₁R₂N-COCl ausgeführt wird, wobei R₁ und R₂ nach einem der vorhergehenden Ansprüche definiert sind.

7. Verfahren nach Anspruch 6, wobei Schritt (a) in Anwesenheit einer Base ausgeführt wird.

8. Verfahren nach Anspruch 6 oder 7, wobei Schritt (a) in Anwesenheit von K₂CO₃ oder Triethylamin ausgeführt wird.

9. Verfahren nach einem der vorhergehenden Ansprüche, wobei Schritt (b) unter Einsatz von Amin HNR₃R₄ ausgeführt wird, wobei R₃ und R₄ nach einem der vorhergehenden Ansprüche definiert sind.

10. Verfahren nach Anspruch 9, wobei Schritt (b) unter Einsatz von reduzierender Aminierung der Carbonylgruppe von Keton (3) mit Amin HNR₃R₄ ausgeführt wird.

11. Verfahren nach Anspruch 9 oder 10, wobei Schritt (b) in Anwesenheit von NaCNBH₃ ausgeführt wird.

12. Verfahren nach einem der Ansprüche 1 bis 9, wobei Schritt (b) durch Reduktion der Carbonylgruppe von Keton (3) ausgeführt wird, um einen Phenylcarbamatalkohol herzustellen, gefolgt von einer Umwandlung des Phenylcarbamatalkohols in ein Phenylcarbamat mit einer Abgangsgruppe, wiederum gefolgt von der Umwandlung des Phenylcarbamats mit der Abgangsgruppe in das Aminoalkylphenylcarbamat mit der Formel (4).

13. Verfahren nach einem der vorhergehenden Ansprüche, ferner den folgenden Schritt umfassend:
(c) Trennen des Aminoalkylphenylcarbamats mit der Formel (4), um ein Aminoalkylphenylcarbamat mit der Formel (5) oder (10) zu erhalten: 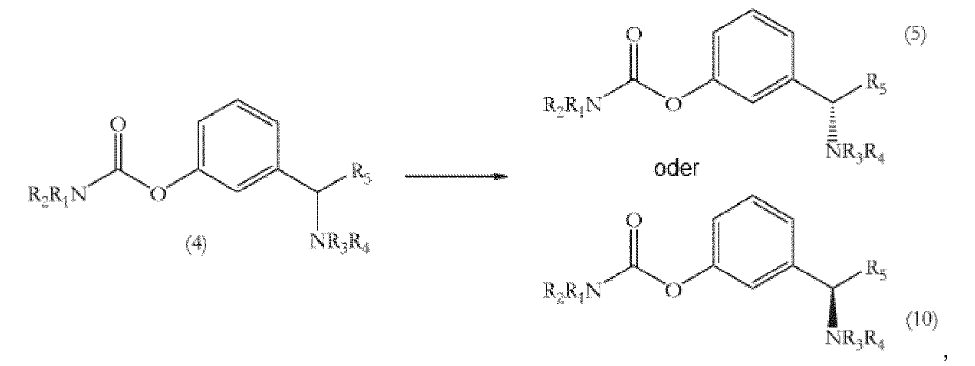 wobei R₁, R₂, R₃, R₄ und R₅ nach einem der vorhergehenden Ansprüche definiert sind und wobei das erhaltene Isomer mit der Formel (5) oder (10) weniger als 5 % andere Isomere derselben Verbindung enthält.

14. Verfahren nach Anspruch 13, wobei die Trennung unter Einsatz einer enantiomerreinen Säure ausgeführt wird, um ein bezogen auf ein oder mehrere andere Isomere bevorzugtes Isomer von Aminoalkylphenylcarbamat (4) zu kristallisieren.

15. Verfahren nach Anspruch 13 oder 14, wobei die Trennung unter Einsatz von (+)-di-O-p-Toluyl-Weinsäure (DPTTA) ausgeführt wird, um ein bezogen auf ein oder mehrere andere Isomere bevorzugtes Isomer von Aminoalkylphenylcarbamat (4) zu kristallisieren

16. Verfahren nach einem der vorhergehenden Ansprüche, ferner umfassend den Schritt des Ausbilden eines Salzes mit der Formel (4s), (5s) oder (10s) des Aminoalkylphenylcarbamats mit der Formel (4), (5) oder (10): 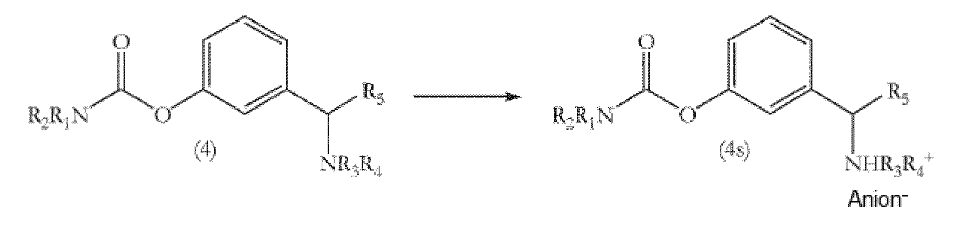 oder 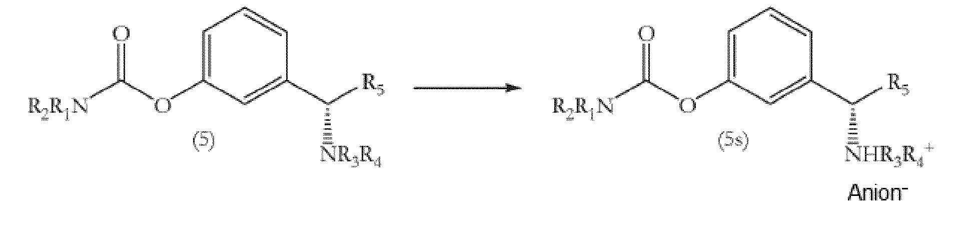 oder 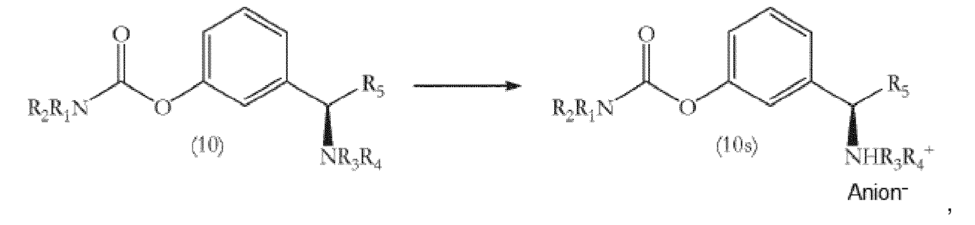 wobei R₁, R₂, R₃, R₄ und R₅ nach einem der vorhergehenden Ansprüche definiert sind und wobei Anion⁻ das Anion einer pharmazeutisch unbedenklichen Säure ist.

17. Verfahren nach Anspruch 16, wobei das Anion ein Anion von Folgendem ist:
(a) Fluss-, Salz-, Bromwasserstoff-, lodwasserstoff-, Salpeter-, Perchlor-, Schwefel-, Phosphor-, Propion-, Butter-, Glycol-, Milch-, Mandel-, Citronen-, Essig-, Benzoe-, Salicyl-, Bernstein-, Äpfel- oder Hydroxybernstein-, Wein-, Fumar-, Malein-, Hydroxymalein-, Schleim- oder Galactar-, Glucon-, Pantothen-, Embon-, Methansulfon-, Trifluormethansulfon-, Ethansulfon-, 2-Hydroxyethansulfon-, Benzensulfon-, Toluen-p-sulfon-, Naphthalen-2-sulfon-, Camphersulfon-, Ornithin-, Glutamin- oder Asparaginsäure; oder
(b) Essig-, Salicyl-, Fumar-, Phosphor-, Sulfon-, Malein-, Bernstein-, Citronen-, Wein-, Proprion- oder Buttersäure; oder
(c) Weinsäure.

18. Verfahren nach einem der vorhergehenden Ansprüche, wobei das Reaktionsgemisch in Schritt (a) weniger als 0.5 molare Äquivalente von NaH aufweist.

19. Verfahren nach einem der vorhergehenden Ansprüche, wobei das Aminoalkylphenylcarbamat (4), (5) oder (10) oder ein pharmazeutisch unbedenkliches Salz davon in Losgrößen ab 5 kg hergestellt wird.

20. Verfahren nach einem der vorhergehenden Ansprüche zur Herstellung von Rivastigmin oder Rivastigminhydrogentartrat.

## Revendications

1. Procédé de préparation d'un phénylcarbamate d'aminoalkyle de formule (4) : 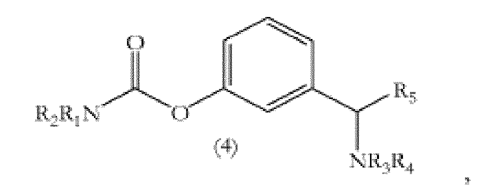 où R₁, R₂, R₃, R₄ et R₅ sont identiques ou différents, chacun étant de l'hydrogène ou un groupe alkyle, alcényle, alcynyle, aryle, arylalkyle, arylalcényle, arylalcynyle, alkylaryle, alcénylaryle ou alcynylaryle, chacun d'eux pouvant être facultativement substitué, et chacun d'eux pouvant facultativement comprendre un ou plusieurs hétéroatomes N, O ou S dans son squelette carbone, ou où -NR₁R₂ ensemble et/ou -NR₃R₄ forment conjointement un groupe cyclohétéroaryle, cyclohétéroalcényle, hétéroaryle, arylcyclohétéroaryle, arylcyclohétéroalcényle, alkylhétéroaryle, alcénylhétéroaryle ou alcynylhétéroaryle, chacun d'eux pouvant être facultativement substitué, et chacun d'eux pouvant facultativement comprendre un ou plusieurs autres hétéroatomes N, O ou S dans son squelette carbone,
le procédé comprenant les étapes consistant à :
(a) convertir une hydroxyphénylcétone de formule (2) en une cétone de phénylcarbamate de formule (3) : 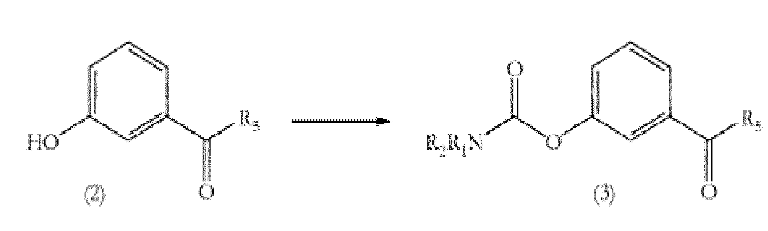 et
(b) convertir la cétone de phénylcarbamate de formule (3) en phénylcarbamate d'aminoalkyle de formule (4) :

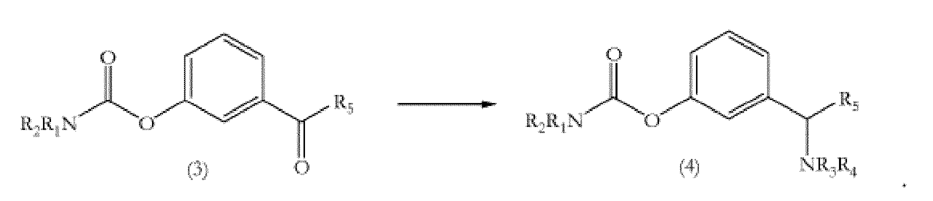

2. Procédé selon la revendication 1, où :
(a) aucun de R₁, R₂, R₃, R₄ et R₅ n'est substitué ; et/ou
(b) aucun de R₁, R₂, R₃, R₄ et R₅ ne comprend d'hétéroatomes dans son squelette carbone.

3. Procédé selon la revendication 1 ou 2, où :
(a) R₁, R₂, R₃, R₄ et R₅ sont identiques ou différents, chacun étant de l'hydrogène ou un groupe alkyle ; ou
(b) R₁, R₂, R₃, R₄ et R₅ sont identiques ou différents, chacun étant de l'hydrogène ou un groupe alkyle en C₁₋₆ ; ou
(c) R₁, R₂, R₃, R₄ et R₅ sont identiques ou différents, chacun étant de l'hydrogène ou un groupe alkyle en C₁₋₃ ; ou
(d) R₁, R₂, R₃, R₄ et R₅ sont identiques ou différents, chacun étant de l'hydrogène, du méthyle ou de l'éthyle.

4. Procédé selon l'une quelconque des revendications précédentes, où R₁, R₂, R₃, R₄ et R₅ sont identiques ou différents, chacun étant un groupe alkyle.

5. Procédé selon la revendication 4, où R₁ est de l'éthyle et R₂, R₃, R₄ et R₅ sont chacun du méthyle.

6. Procédé selon l'une quelconque des revendications précédentes, où l'étape (a) est effectuée en utilisant du chlorure de carbamoyle R₁R₂N-OCl, où R₁ et R₂ sont tels que définis dans l'une quelconque des revendications précédentes.

7. Procédé selon la revendication 6, où l'étape (a) est effectuée en présence d'une base.

8. Procédé selon la revendication 6 ou 7, où l'étape (a) est effectuée en présence de K₂CO₃ ou de triéthylamine.

9. Procédé selon l'une quelconque des revendications précédentes, où l'étape (b) est effectuée en utilisant de l'amine HNR₃R₄, où R₃ et R₄ sont tels que définis dans l'une quelconque des revendications précédentes.

10. Procédé selon la revendication 9, où l'étape (b) est effectuée par amination réductrice du groupe carbonyle de la cétone (3) avec l'amine HNR₃R₄.

11. Procédé selon la revendication 9 ou 10, où l'étape (b) est effectuée en présence de NaCNBH₃.

12. Procédé selon l'une quelconque des revendications 1 à 9, où l'étape (b) est effectuée par réduction du groupe carbonyle de la cétone (3) pour produire un alcool de phénylcarbamate, puis par conversion de l'alcool de phénylcarbamate en un phénylcarbamate portant un groupe partant, puis par conversion du phénylcarbamate portant un groupe partant en phénylcarbamate d'aminoalkyle de formule (4).

13. Procédé selon l'une quelconque des revendications précédentes, comprenant en outre l'étape consistant à :
(c) dissoudre le phénylcarbamate d'aminoalkyle de formule (4) pour obtenir un phénylcarbamate d'aminoalkyle de formule (5) ou (10) : 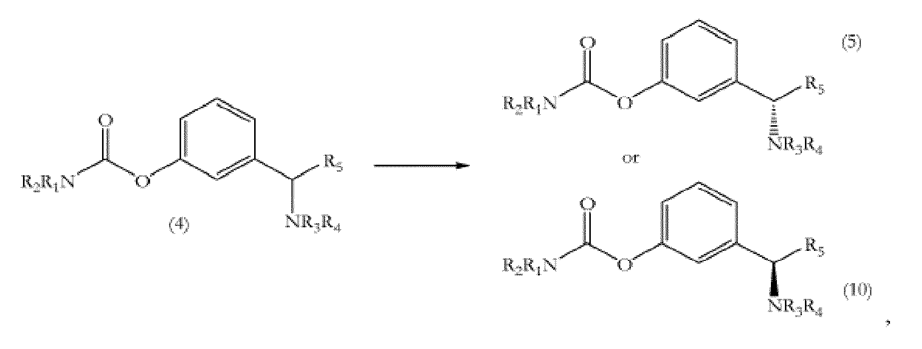 où R₁, R₂, R₃, R₄ et R₅ sont tels que définis dans l'une quelconque des revendications précédentes, et où l'isomère de formule (5) ou (10) obtenu comprend moins de 5 % d'autres isomères du même composé.

14. Procédé selon la revendication 13, où la dissolution est effectuée en utilisant un acide énantiomériquement pur pour cristalliser un isomère de phénylcarbamate d'aminoalkyle (4) plutôt qu'un ou plusieurs autres isomères.

15. Procédé selon la revendication 13 ou 14, où la dissolution est effectuée en utilisant de l'acide (+)-di-O-p-toluyltartrique (DPTTA) pour cristalliser un isomère de phénylcarbamate d'aminoalkyle (4) plutôt qu'un ou plusieurs autres isomères.

16. Procédé selon l'une quelconque des revendications précédentes, comprenant en outre l'étape consistant à former un sel de formule (4s), (5s) ou (10s) du phénylcarbamate d'aminoalkyle de formule (4), (5) ou (10) : 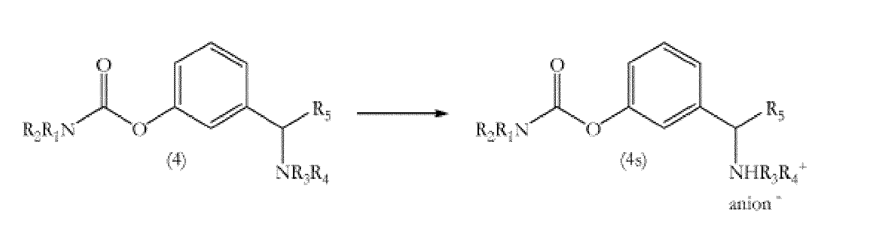 or 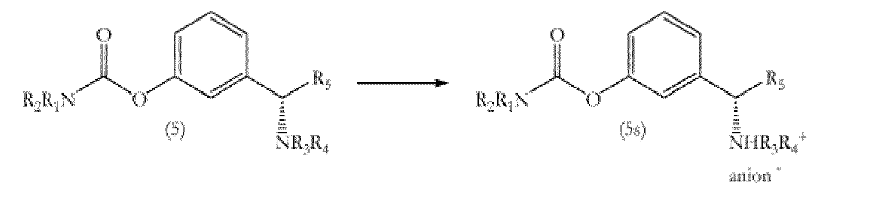 or 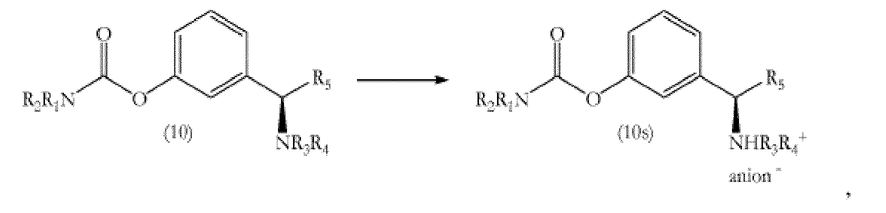 où R₁, R₂, R₃, R₄ et R₅ sont tels que définis dans l'une quelconque des revendications précédentes, et où l'anion- est l'anion d'un quelconque acide pharmaceutiquement acceptable.

17. Procédé selon la revendication 16, où l'anion est un anion de :
(a) l'acide fluorhydrique, chlorhydrique, bromhydrique, iodhydrique, nitrique, perchlorique, sulfurique, phosphorique, propionique, butyrique, glycolique, lactique, mandélique, citrique, acétique, benzoïque, salicylique, succinique, malique ou hydroxysuccinique, tartrique, fumarique, maléique, hydroxymaléique, mucique ou galactarique, gluconique, pantothénique, pamoïque, méthanesulfonique, trifluorométhanesulfonique, éthanesulfonique, 2-hydroxyéthanesulfonique, benzènesulfonique, toluène-p-sulfonique, naphtalène-2-sulfonique, camphorsulfonique, ornithinique, glutamique ou aspartique ; ou
(b) l'acide acétique, salicylique, fumarique, phosphorique, sulfonique, maléique, succinique, citrique, tartrique, propionique ou butyrique ; ou
(c) l'acide tartrique.

18. Procédé selon l'une quelconque des revendications précédentes, où le mélange réactionnel de l'étape (a) comprend moins de 0,5 équivalent molaire de NaH.

19. Procédé selon l'une quelconque des revendications précédentes, où le phénylcarbamate d'aminoalkyle (4), (5) ou (10), ou son sel pharmaceutiquement acceptable, est produit en lots de 5 kg ou plus.

20. Procédé selon l'une quelconque des revendications précédentes, pour la préparation de rivastigamine ou d'hydrogénotartrate de rivastigamine.
